(19) Europäisches Patentamt
European Patent Office
Office européen des brevets

(11) Veröffentlichungsnummer: **0 019 134**
**B1**

(12) # EUROPÄISCHE PATENTSCHRIFT

(45) Veröffentlichungstag der Patentschrift: **28.07.82**

(51) Int. Cl.³: **C 07 D 233/60**, A 01 N 43/50

(21) Anmeldenummer: **80102293.0**

(22) Anmeldetag: **28.04.80**

(54) **Fluorierte 1-Imidazolyl-butan-Derivate, Verfahren zu ihrer Herstellung und ihre Verwendung als Fungizide.**

(30) Priorität: **10.05.79 DE 2918893**

(43) Veröffentlichungstag der Anmeldung: **26.11.80 Patentblatt 80/24**

(45) Bekanntmachung des Hinweises auf die Patenterteilung: **28.07.82 Patentblatt 82/30**

(84) Benannte Vertragsstaaten: **AT BE CH DE FR GB IT LI NL SE**

(56) Entgegenhaltungen:
**EP-A-0 009 707**
**DE-A-2 105 490**
**DE-A-2 325 156**
**DE-A-2 632 601**
**DE-A-2 811 916**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal-1 (DE)**
Erfinder: **Büchel, Karl Heinz, Dr., Bergerheide 62, D-5600 Wuppertal-1 (DE)**
Erfinder: **Stetter, Jörg, Dr., Gellertweg 4, D-5600 Wuppertal-1 (DE)**
Erfinder: **Frohberger, Paul-Ernst, Dr., Willi-Baumeister-Strasse 5, D-5090 Leverkusen (DE)**
Erfinder: **Brandes, Wilhelm, Dr., Eichendorffstrasse 3, D-5623 Leichlingen (DE)**
Erfinder: **Scheinpflug, Hans, Dr., Am Thelenhof 15, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 134 B1

ACTORUM AG

# Fluorierte 1-Imidazolylbutanderivate, Verfahren zu ihrer Herstellung und ihre Verwendung als Fungizide

Die Erfindung betrifft neue fluorierte 1-Imidazolbutanderivate, ein Verfahren zu ihrer Herstellung und ihre Verwendung als Fungizide.

Es ist bereits bekannt geworden, dass chlorierte und bromierte 1-Imidazolylbutanderivate gute fungizide Eigenschaften aufweisen (vergl. DE-OS Nr. 2632602).

Die Wirkung dieser Verbindungen ist jedoch, insbesondere bei niedrigen Aufwandmengen und -konzentrationen, nicht immer ganz befriedigend.

Es wurden neue fluorierte 1-Imidazolylbutanderivate der allgemeinen Formel I

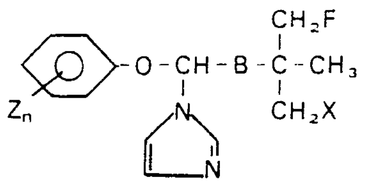

(I)

in welcher

B für die Ketogruppe oder die CH(OH)-Gruppierung steht,

X für Wasserstoff oder Fluor steht,

Z für Halogen, Alkyl mit 1 bis 4 C-Atomen, Nitro, Cyano, Alkoxycarbonyl mit 1 bis 4 C-Atomen im Alkylteil oder gegebenenfalls durch Halogen substituiertes Phenyl steht und

n für 0, 1, 2 oder 3 steht,

sowie deren Säureaddukte und Metallsalzkomplexe gefunden.

Diejenigen Verbindungen der allgemeinen Formel I, in welchen B für die CH(OH)-Gruppierung steht, besitzen zwei asymmetrische Kohlenstoffatome; sie können deshalb in den beiden geometrischen Isomeren (threo- und erythro-Form) vorliegen, die in unterschiedlichen Mengenverhältnissen anfallen können. In beiden Fällen liegen sie als optische Isomere vor. Sämtliche Isomeren werden erfindungsgemäss beansprucht.

Weiterhin wurde gefunden, dass man die fluorierten 1-Imidazolylbutanderivate der allgemeinen Formel I erhält, wenn man in an sich bekannter Weise Halogenätherketone der allgemeinen Formel II

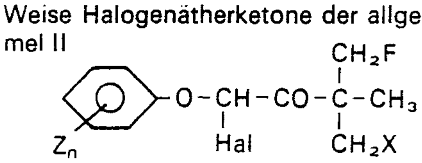

(II)

in welcher

X, Z und n die oben angegebene Bedeutung haben und

Hal für Halogen, vorzugsweise Chlor oder Brom, steht,

mit Imidazol in Gegenwart eines Säurebindemittels und gegebenenfalls in Gegenwart eines Verdünnungsmittels umsetzt und

gegebenenfalls noch die erhaltenen Ketoderivate nach bekannten Methoden in üblicher Weise reduziert.

An die so erhaltenen Verbindungen der allgemeinen Formel I können gegebenenfalls anschliessend eine Säure oder ein Metallsalz addiert werden.

Die neuen fluorierten 1-Imidazolylbutanderivate weisen starke fungizide Eigenschaften auf. Dabei zeigen überraschenderweise die erfindungsgemässen Verbindungen eine erheblich höhere Wirkung als die aus dem Stand der Technik bekannten chlorierten und bromierten 1-Imidazolbutanderivate, die chemisch und wirkungsmässig naheliegendste Verbindungen sind.

Die erfindungsgemässen Stoffe stellen somit eine Bereicherung der Technik dar.

Die erfindungsgemässen fluorierten 1-Imidazolylbutanderivate sind durch die allgemeine Formel I definiert.

Bevorzugt sind diejenigen fluorierten 1-Imidazolylbutanderivate, in denen Z für Halogen, Methyl, Äthyl, Nitro, Cyano, Methoxycarbonyl, Äthoxycarbonyl, Phenyl oder Chlorphenyl steht und der Index n für 0, 1 oder 2 steht.

Im einzelnen seien ausser den bei den Herstellungsbeispielen genannten Verbindungen die folgenden Verbindungen der allgemeinen Formel I genannt:

| $Z_n$ | X | B |
|---|---|---|
| — | H | CO |
| 2-F | H | CO |
| 3-F | H | CO |
| 4-F | H | CO |
| 2-Cl | H | CO |
| 3-Cl | H | CO |
| 2-Br | H | CO |
| 3-Br | H | CO |
| $2-CH_3$ | H | CO |
| $4-CH_3$ | H | CO |
| 2- 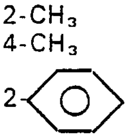 | H | CO |
| 4- 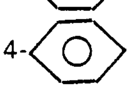 | H | CO |
| 4- 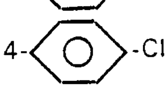 | H | CO |
| $2-NO_2$ | H | CO |
| 4-CN | H | CO |
| $4-COOCH_3$ | H | CO |
| $4-COOC_2H_5$ | H | CO |
| 4-J | H | CO |
| $4-Cl,2-CH_3$ | H | CO |
| $4-CH_3,2-Cl$ | H | CO |
| — | H | CH(OH) |
| 2-F | H | CH(OH) |
| 3-F | H | CH(OH) |
| 4-F | H | CH(OH) |
| 2-Cl | H | CH(OH) |
| 3-Cl | H | CH(OH) |
| 2-Br | H | CH(OH) |
| 3-Br | H | CH(OH) |
| $2-CH_3$ | H | CH(OH) |

| $Z_n$ | X | B |
|---|---|---|
| 4-$CH_3$ | H | CH(OH) |
| 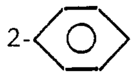 | H | CH(OH) |
| 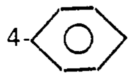 | H | CH(OH) |
| 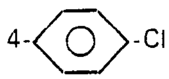 | H | CH(OH) |
| 2-$NO_2$ | H | CH(OH) |
| 4-CN | H | CH(OH) |
| 4-$COOCH_3$ | H | CH(OH) |
| 4-$COOC_2H_5$ | H | CH(OH) |
| 4-J | H | CH(OH) |
| 4-Cl,2-$CH_3$ | H | CH(OH) |
| 4-$CH_3$,2-Cl | H | CH(OH) |
| — | F | CO |
| 2-F | F | CO |
| 3-F | F | CO |
| 3-Cl | F | CO |
| 2-Br | F | CO |
| 3-Br | F | CO |
| 4-Br | F | CO |
| 2-$CH_3$ | F | CO |
| 4-$CH_3$ | F | CO |
| 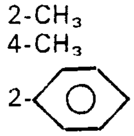 | F | CO |
| 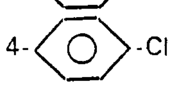 | F | CO |
| 2-$NO_2$ | F | CO |
| 4-$NO_2$ | F | CO |
| 4-CN | F | CO |
| 4-$COOCH_3$ | F | CO |
| 4-$COOC_2H_5$ | F | CO |
| 4-J | F | CO |
| 4-Cl,2-$CH_3$ | F | CO |
| — | F | CH(OH) |
| 2-F | F | CH(OH) |
| 3-F | F | CH(OH) |
| 3-Cl | F | CH(OH) |
| 2-Br | F | CH(OH) |
| 3-Br | F | CH(OH) |
| 4-Br | F | CH(OH) |
| 2-$CH_3$ | F | CH(OH) |
| 4-$CH_3$ | F | CH(OH) |
| 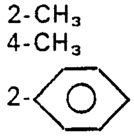 | F | CH(OH) |
| 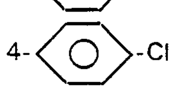 | F | CH(OH) |
| 2-$NO_2$ | F | CH(OH) |
| 4-$NO_2$ | F | CH(OH) |
| 4-CN | F | CH(OH) |
| 4-$COOCH_3$ | F | CH(OH) |
| 4-$COOC_2H_5$ | F | CH(OH) |
| 4-J | F | CH(OH) |
| 4-Cl,2-$CH_3$ | F | CH(OH) |
| 4-$CH_3$,2-Cl | F | CH(OH) |
| 4-F | F | CH(OH) |

Verwendet man beispielsweise 1-Brom-1-(4-chlorphenoxy)-3,3-dimethyl-4-fluorbutan-2-on und Imidazol als Ausgangsstoffe, so kann der Reaktionsablauf durch das folgende Formelschema wiedergegeben werden:

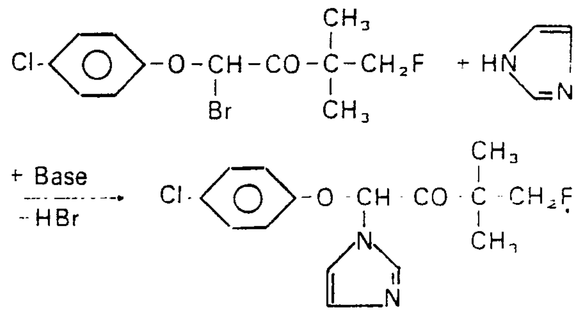


Verwendet man 1-(4-Chlorphenoxy)-3,3-dimethyl-4-fluor-1-(imidazol-1-yl)butan-2-on und Natriumborhydrid als Ausgangsstoffe so kann der Reaktionsablauf der Reduktion durch das folgende Formelschema wiedergegeben werden:

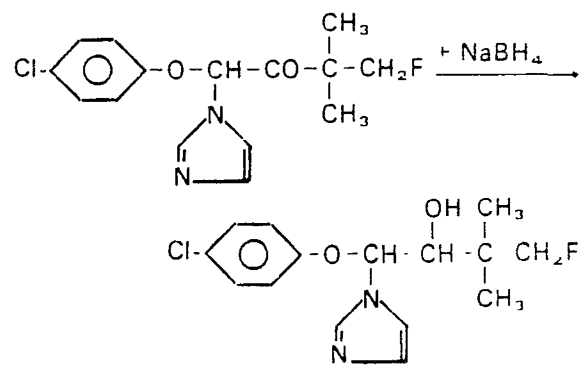


Die bei der Durchführung des erfindungsgemässen Verfahrens als Ausgangsstoffe zu verwendenden Halogenatherketone sind durch die allgemeine Formel II definiert. In dieser Formel stehen X, Z und der Index n vorzugsweise für diejenigen Reste, die bereits im Zusammenhang mit der Beschreibung der erfindungsgemässen Stoffe der allgemeinen Formel I vorzugsweise fur diese Substituenten genannt wurden.

Die Halogenätherketone der allgemeinen Formel II sind noch nicht bekannt. Sie können jedoch nach bekannten Verfahren (vergleiche z.B. DE-OS Nr. 2632603 erhalten werden, indem man z.B. bekannte Phenole der allgemeinen Formel III

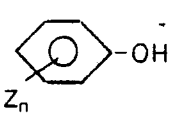 (III)

in welcher

Z und n die oben angegebene Bedeutung haben,

mit einem Halogenketon der allgemeinen Formel IV

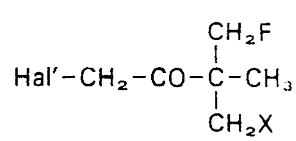 (IV)

in welcher

X die oben angegebene Bedeutung hat und

Hal' für Chlor oder Brom steht,

umsetzt. Das noch verbliebene aktive Wasserstoffatom wird anschliessend in üblicher Weise gegen Halogen ausgetauscht (vergl. auch die Herstellungsbeispiele).

Die Halogenketone der allgemeine Formel IV sind ebenfalls noch nicht bekannt. Sie können jedoch auf allgemein übliche und bekannte Weise erhalten werden, indem man Fluorderivate des 3.3-Dimethylbutan-2-ons der allgemeinen Formel V

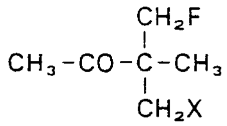 (V)

in welcher

X die oben angegebene Bedeutung hat,

mit Chlor oder Brom in Gegenwart eines inerten organischen Lösungsmittels, wie beispielsweise Äther oder chlorierte Kohlenwasserstoffe, bei Raumtemperatur versetzt (vergl. auch die Herstellungsbeispiele), oder mit üblichen Chlorierungsmitteln,wie beispielsweise Sulfurylchlorid bei 20 bis 60°C umsetzt.

Die Fluorderivate des 3,3-Dimethylbutan-2-ons der allgemeinen Formel V sind Gegenstand einer eigenen älteren Patentanmeldung (vergl. DE-OS Nr. 2843767). Man erhält die Verbindungen der allgemeinen Formel V, wenn man Sulfonsäureester der allgemeinen Formel VI

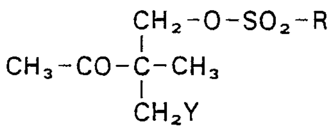 (VI)

in welcher

R für Alkyl mit 1 bis 4 Kohlenstoffatomen, insbesondere Methyl, oder Aryl mit 6 bis 12 Kohlenstoffatomen, insbesondere Phenyl oder Tolyl, steht und

Y für Wasserstoff oder die Gruppe $-O-SO_2-R$ steht,

mit Metallfluoriden, wie beispielsweise Natrium- und Kaliumfluorid, in Gegenwart eines polaren organischen Lösungsmittels, wie beispielsweise Di-, Tri- oder Tetraäthylenglykol, bei Temperaturen zwischen 80 und 250°C umsetzt (vgl. auch die Herstellungsbeispiele).

Die Sulfonsäureester der allgemeinen Formel VI sind teilweise bekannt [J. Org. Chem.*35*, 2391 (1970)]. Die noch nicht beschriebenen Verbindungen können nach literaturbekannten Verfahren aus den entsprechenden Hydroxybutanonen und Sulfochloriden in Gegenwart von Basen hergestellt werden (siehe z.B. Houben-Weyl, „Methoden der Org. Chemie", Bd IX, S. 388 u. 663, sowie die Angaben bei den Herstellungsbeispielen).

Für die erfindungsgemässe Umsetzung kommen als Verdünnungsmittel inerte organische Lösungsmittel infrage. Hierzu gehören vorzugsweise Ketone, wie Diethylketon und insbesondere Aceton und Methyläthylketon; Nitrile, wie Propionitril, insbesondere Acetonitril; Alkohole, wie Äthanol oder Isopropanol; Äther, wie Tetrahydrofuran oder Dioxan; Benzol; Toluol; Formamide, wie insbesondere Dimethylformamid; und halogenierte Kohlenwasserstoffe.

Die erfindungsgemässe Umsetzung wird in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan.

Vorzugsweise verwendet man einen entsprechenden Überschuss an Imidazol.

Die Reaktionstemperaturen können in einem grösseren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmässigerweise beim Siedepunkt des jeweiligen Lösungsmittels gearbeitet.

Bei der Durchführung des erfindungsgemässen Verfahrens setzt man auf 1 mol der Verbindungen der allgemeinen Formel II vorzugsweise 2 mol Imidazol und 1 bis 2 mol Säurebinder ein. Zur Isolierung der Verbindungen der allgemeinen Formel I wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw. Umkristallisation oder Salzbildung und Umkristallisation gereinigt.

Die erfindungsgemässe Reduktion erfolgt in üblicher Weise, wie z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemässe Umsetzung polare organische Lösungsmittel infrage. Hierzu gehören vorzugsweise Alkohole, wie Methanol, Äthanol, Butanol, Isopropanol, und Äther, wie Diethyläther oder Tetrahydrofuran. Die Reaktion wird im allgemeinen bei 0 bis 30°C, vorzugsweise bei 0 bis 20°C durchgeführt. Hierzu setzt man auf 1 mol des Ketons der allgemeinen Formel I etwa 1 mol eines komplexen Hydrids, wie Natriumborhydrid oder Lithiumalanat, ein. Zur Isolierung der reduzierten Verbindungen der allgemeinen Formel I wird der Rückstand in verdünnter Salzsäure aufgenommen, anschliessend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemässe Umsetzung bevorzugt Alkohole, wie Isopropanol, oder inerte Kohlenwasserstoffe, wie Benzol, infrage. Die Reaktionstemperaturen können wiederum in einem grösseren Bereich variiert werden; im allgemeinen arbeitet man zwischen 20 und 120°C, vorzugsweise bei 50 bis 100°C. Zur Durchführung der Reaktion setzt man auf 1 mol des Ketons der allgemeinen Formel I etwa 0,3 bis 2 mol Aluminiumisopropylat ein. Zur Isolierung der reduzierten Verbindungen der allgemeinen Formel I wird das überschüssige Lösungsmittel im Vakuum entfernt und die entstandenen Aluminiumverbindungen mit verdünnter Schwefelsäure oder Natronlauge zersetzt. Die weitere Aufarbeitung erfolgt in üblicher Weise.

Zur Herstellung von Säureadditionssalzen der Verbindungen der allgemeinen Formel I kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäuren, wie z.B. p-Toluolsulfonsäure und 1,5-Naphthalindisulfonsäure.

Die Salze der Verbindungen der allgemeinen Formel I können in einfacher Weise nach üblichen Salzbindungsmethoden, z.B. durch Lösen einer Verbindung in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel oder durch Umkristallisation gereinigt werden.

Zur Herstellung von Metallsalzkomplexen der Verbindungen der allgemeinen Formel I kommen vorzugsweise Salze von Metallen der II. bis IV.-Haupt- und der I. und II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der allgemeinen Formel I können in einfacher Weise nach üblichen Verfahren erhalten werden, so z.B. durch Lösen des Metallsalzes in Alkohol, z.B. Äthanol, und Hinzufügen zur Verbindung. Man kann Metallsalzkomplexe in bekannter Weise, z.B. durch Abfiltrieren, isolieren und gegebenenfalls durch Umkristallisieren reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung des Getreidemehltaus (Erysiphe cichoracearum) sowie gegen Getreidekrankheiten, wie gegen Getreidemehltau, Getreiderost und Gerstenmehltau, eingesetzt werden.

Hervorzuheben ist, dass die erfindungsgemässen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in an sich bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylenfettsäureester, Polyoxyäthylenfettalkoholäther, z.B. Alkylarylpolyglykoläther, Alkylsulfonate, Alkylsulfate, Arylsulfonate sowie Einweisshydrolysate; als Dispergiermittel kommen in Frage: z.B. Ligninsulfatablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90 Gew.%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trokkenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.%, vorzugsweise zwischen 0,5 und 0,001 Gew.%.

Bei der Saatgutbehandlung werden im allgemeinen Wirkstoffmengen von 0,001 bis 50 g je kg Saatgut, vorzugsweise 0,01 bis 10 g, benötigt.

Bei Behandlung des Bodens sind Wirkstoffkonzentrationen von 0,00001 bis 0,1 Gew.%, vorzugsweise von 0,0001 bis 0,02 Gew.%, am Wirkungsort erforderlich.

In den nachfolgenden Beispielen werden die nachstehend angegebenen Verbindungen als Vergleichssubstanzen eingesetzt:

(A) 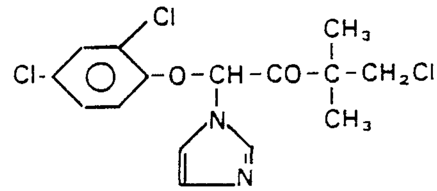

(B) 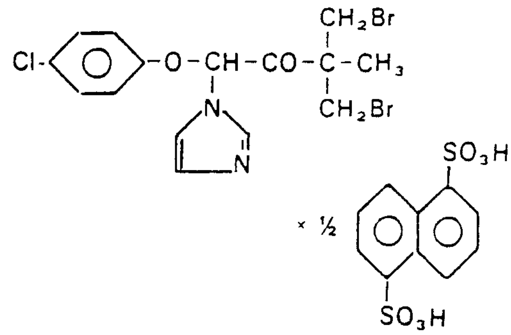

× ½

(C) 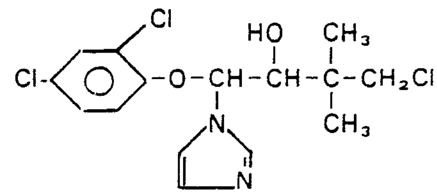

(D) 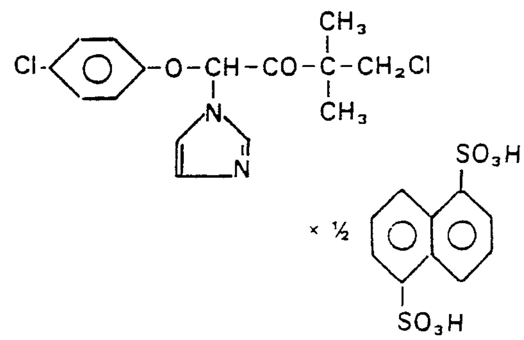

× ½

(E) 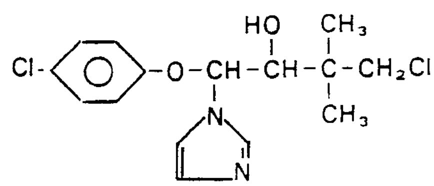

*Beispiel A*

*Sprossbehandlungstest/Getreidemehltau/ Protektiv (blattzerstörende Mykose)*

Zur Herstellung einer zweckmässigen Wirkstoffzubereitung nimmt man 0,25 Gewichsteile Wirkstoff in 25 Gewichtsteilen Dimethylformamid und 0,06 Gewichtsteilen Emulgator (Alkylarylpolyglykoläther) auf und gibt 975 Gewichsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var.hordei.

Nach 6 d Verweilzeit der Pflanzen bei einer Temperatur von 21 bis 22°C und einer Luftfeuchtigkeit von 80 bis 90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Mehltaubefall ist.

Bei diesem Test zeigen z.B. die folgenden Verbindungen eine sehr gute Wirkung, die derjenigen der aus dem Stand der Technik bekannten Verbindungen (A) und (B) überlegen ist:
Verbindungen gemäss Herstellungsbeispielen 1,8,4,9,6 und 2.

*Beispiel B*

*Gerstenmehltautest (Erysiphe graminis var.hordei)/systemisch (pilzliche Getreidesprosskrankheit)*

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3×12 Korn in Blumentöpfe 2 cm tief in ein Gemisch aus einem Volumenteil Fruhstorfer Einheitserde und einem Volumenteil Quarzsand ein. die Keimung und der Auflauf erfolgen unter günstigen Bedingungen im Gewächshaus. 7 d nach der Aussaat, wenn die Gerstenpflanzen ihr erstes Blatt entfaltet graminis var.hordei bestäubt und bei 21 bis 22°C und 80 bis 90% rel. Luftfeuchte und 16stündiger Belichtung weiter kultiviert. Innerhalb von 6 d bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Bei diesem Test zeigen z.B. die folgenden Verbindungen eine sehr gute Wirkung, die derjenigen der aus dem Stand der Technik bekannten Verbindungen (A), (B) und (C) überlegen ist:
Verbindungen gemäss Herstellungsbeispielen: 1,2,8,4,5 und 6.

*Beispiel C*

*Sprossbehandlungstest/Getreiderost/protektiv (blattzerstörende Mykose)*

Zur Herstellung einer zweckmässigen Wirkstoffzubereitung nimmt man 0,25 Gewichtsteile Wirkstoff in 25 Gewichtsteilen Dimethylformamid und 0,06 Gewichtsteilen Emulgator Alkylarylpolyglykoläther auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 h bei etwa 20°C und einer 100%igen Luftfeuchtigkeit in ein Gewächshaus.

Nach 10 d Verweilzeit der Pflanzen bei einer Temperatur von 20°C und einer Lufteuchtigkeit von 80 bis 90% wertet man den Besatz der Pflanzen mit Rostpusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Rostbefall ist.

In diesem Test zeigen z.B. die folgenden Verbindungen eine sehr gute Wirkung, die derjenigen der aus dem Stand der Technik bekannten Verbindungen (D) und (E) überlegen ist:
Verbindungen gemäss Herstellungsbeispielen 5 und 10.

*Beispiel D*

*Erysiphe-Test (Gurken)/Protektiv*

Lösungsmittel:
4,7 Gewichtsteile Aceton
Emulgator:
0,3 Gewichtsteile Alkylarylpolyglykoläther
Wasser:
95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 h im Gewächshaus. Dann werden sie zur Inokulation mit Konidien des Pilzes Erysiphe cichoracearum bestäubt. Die Pflanzen werden anschliessend bei 23 bis 24°C und bei einer relativen Lufteuchtigkeit von ca 75% im Gewächshaus aufgestellt.

Nach 12 d wird der Befall der Gurkenpflanzen bestimmt. Die erhaltenen Boniturwerte werden in Prozent Befall umgerechnet. 0% bedeutet keinen Befall, 100% bedeutet, dass die Pflanzen vollständig befallen sind.

Bei diesem Test zeigen z.B. die folgenden Verbindungen eine sehr gute Wirkung, die derjenigen der aus dem Stand der Technik bekannten Verbindung (D) überlegen ist:
Verbindungen gemäss Herstellungsbeispielen: 10.1,8 und 4.

*Beispiel 1*

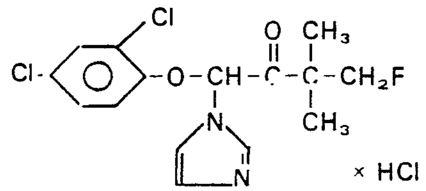

157,5 g (0,44 mol) 1-Brom-1-(2,4-dichlorphenoxy)-3,3-dimethyl-4-fluor-2-butanon werden in 500 ml Acetonitril gelöst und zu einer Lösung von 109 g (1,6 mol) Imidazol in 600 ml Acetonitril getropft. Man erhitzt 10 h unter Rückfluss. Danach wird ds Lösungsmittel im Wasserstrahlvakuum abdestilliert, der Rückstand in 1000 ml Methylenchlorid aufgenommen und dreimal mit je 500 ml Wasser gewaschen. Die organische Phase wird über Natriumsulfat getrocknet, filtriert und im Wasserstrahlvakuum durch Abdestillieren des Lösungsmittels eingeengt. Der Rückstand wird in 500 ml Äthanol gelöst, mit 40 ml konzentrierter Salzsäure versetzt und das Lösungsmittel im Wasserstrahlvakuum abdestilliert. Der Rückstand wird mit 500 ml Äther verrührt, wobei es zur Kristallisation kommt. Man erhält 66 g (39,3% der Theorie) 1-(2,4-Dichlorphenoxy)-3,3-dimethyl-4-fluor-1-(imidazol-1-yl)-2-butanonhydrochlorid vom Schmelzpunkt 91 bis 110°C.

*Herstellung der Vorstufen*

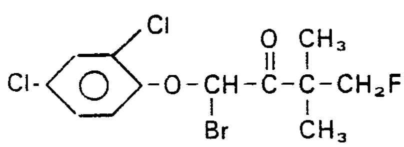

244,6 g (0,88 mol) rohes 1-(2,4-Dichlorphenoxy)-3,3-dimethyl-4-fluor-2-butanon werden in 700 ml Chloroform gelöst und bei 30°C tropfenweise mit 46 ml Brom so versetzt, dass laufend Entfärbung eintritt. Nach beendeter Zugabe lässt man 30 min nachrühren und engt dann durch Abdestillieren des Chloroforms im Vakuum ein. Man erhält 315 g (100% der Theorie) 1-Brom-1(2,4-dichlorphenoxy)-3,3-dimethyl-4-fluor-2-butanon als viskoses Öl, das roh weiter umgesetzt werden kann.

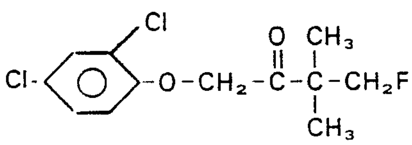

Zu 140 g (1 mol) Kaliumcarbonat und 163 g (1 mol) 2,4-Dichlorphenol in 1000 ml Aceton tropft man in der Siedehitze eine Lösung von 197 g (1 mol) 1-Brom-3,3-dimethyl-4-fluor-2-butanon in 100 ml Aceton. Man lässt 20 h unter Rückfluss rühren, kühlt ab und filtriert. Das Filtrat wird durch Abdestillieren des Acetons im Wasserstrahlvakuum eingeengt. Der Rückstand wird in 500 ml Toluol gelöst, mit 500 ml 10%iger Natronlauge und zweimal mit je 250 ml Wasser gewaschen, über Natriumsulfat getrocknet und durch Abdestillieren des Toluols im Wasserstrahlvakuum eingeengt. Man erhält 244,6 g (88% der Theorie) 1-(2,4-Dichlorphenoxy)-3,3-dimethyl-4-fluor-2-butanon als hochviskoses Öl, das roh weiter umgesetzt werden kann.

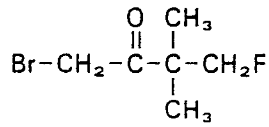

118 g (1 mol) 3,3-Dimethyl-4-fluor-2-butanon werden in 600 ml Chloroform gelöst und tropfenweise so mit 52 ml Brom versetzt, dass laufend Entfärbung eintritt. Nach beendeter Zugabe lässt man 30 min weiterrühren. Danach wird durch Abdestillieren des Lösungsmittels im Vakuum eingeengt. Man erhält 197 g (100% der Theorie) 1-Brom-3,3-dimethyl-4-fluor-2-butanon vom Siedepunkt 85 bis 90°C/12 mm Hg-Säule, das roh weiter umgesetzt werden kann.

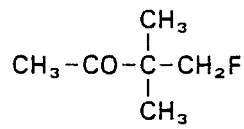

Zu der in einem Dreihals-Rührkolben mit absteigendem Kühler befindlichen Suspension von 23,2 g (0,4 mol) trockenem Kaliumfluorid in 400 ml dest. Teträthylenglykol werden bei 160°C und 20 mbar 38,8 g (0,2 mol) 2,2-Dimethyl-2-oxobutylmethansulfonat im Verlauf von 2 h zugetopft und weitere 2 h nachgerührt. An einem absteigenden Kondensator und in einer nachgeschalteten Tiefkühlfalle wird das abdestillierte Reaktionsprodukt kondensiert und gesammelt. Man erhält 20,9 g (89% der Theorie) 3,3-Dimethyl-4-fluor-2-butanon, vom Siedepunkt 130 bis 134°C.

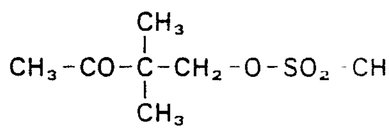

232 g (2 mol) 3,3-Dimethyl-4-hydroxy-2-butanon (z. Herstellung vgl. Beilstein *H 1* E III 3239, IV 4030 und Bull. Soc. Chim. France, *1964*, 2849) werden in 700 ml absolutem Pyridin bei 0 bis 5°C mit 229 g (2 mol) Methansulfochlorid umgesetzt. Nach 12 h Stehen bei 20°C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332 g (86% der Theorie) 2,2-Dimethyl-3-oxobutylmethansulfonat vom Siedepunkt 106 bis 120°C/0,12 mm Hg-Säule.

*Beispiel 2*

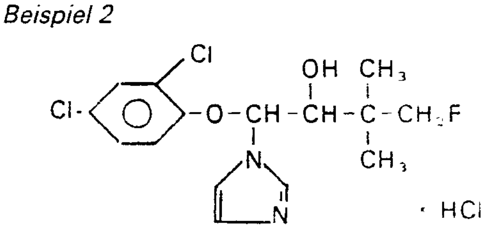

44 g (0,1275 mol) 1-(2,4-Dichlorphenoxy-

3,3-dimethyl-4-fluor-1-(imidazol-1-yl)-2-butanon (Beispiel 1) werden in 300 ml Methanol gelöst und bei 0 bis 5°C mit 6,3 g Natriumborhydrid versetzt. Man lässt 15 h bei Raumtemperatur nachrühren, tropft 60 ml konzentrierte Salzsäure unter Eiskühlung zu, verrührt 10 h bei Raumtemperatur und destilliert das Lösungsmittel im Wasserstrahlvakuum ab. Der Rückstand wird in 250 ml Methylenchlorid aufgenommen, in 500 ml wässrige, gesättigte Natriumhydrogencarbonatlösung eingerührt, die Methylenchloridphase abgetrennt, dreimal mit je 100 ml Wasser gewaschen, die organische Phase über Natriumsulfat getrocknet und das Lösungsmittel abdestilliert. Das zurückbleibende Öl wird in 200 ml Äther aufgenommen, 50 ml ätherische Salzsäure zugegeben und das Lösungsmittel abdestilliert. Man erhält 28,2 g (58% der Theorie) 1-(2,4-Dichlorphenoxy)-3,3-dimethyl-4-fluor-1-(imidazol-1-yl)-2-butanol-hydrochlorid vom Schmelzpunkt 184 bis 210°C als Diastereomerengemisch.

*Beispiel 3*

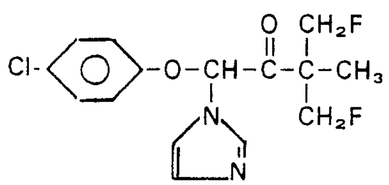

61,5 g (0,18 mol) 3,3-Bisfluormethyl-1-brom-1-(4-chlorphenoxy)butan-2-on werden mit 27,2 g (0,4 mol) Imidazol in 500 ml Acetonitril 4 h bei 45°C gerührt. Das Lösungsmittel wird im Wasserstrahlvakuum abdestilliert, das zurückbleibende Öl in 500 ml Methylenchlorid aufgenommen, die organische Phase zweimal mit 1000 ml Wasser gewaschen, über Natriumsulfat getrocknet und das Lösungsmittel abdestilliert. Das Öl wird in Aceton aufgenommen, mit 36 g (0,1 mol) 1,5-Naphthalindisulfonsäuretetrahydrat versetzt und der entstehende Niederschlag abgesaugt. Der Niederschlag (das 1,5-Naphthalindisulfonat des Beispiels 3) wird mit Natriumhydrogencarbonatlösung behandelt. Man erhält 14 g (24% der Theorie) 3,3-Bisfluormethyl-1-(4-chlorphenoxy)-1-(imidazol-1-yl)butan-2-on als zähflüssiges Öl.

*Herstellung der Vorstufen*

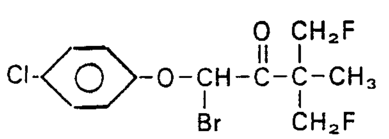

Entsprechend Beispiel 1 durch Umsetzung von 3,3-Bis-fluormethyl-1-(4-chlorphenoxy)butan-2-on mit Brom.

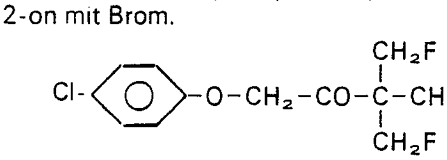

Entsprechend Beispiel 1 durch Umsetzung von 4-Chlorphenol mit 3,3-Bisfluormethyl-1-brombutan-2-on.

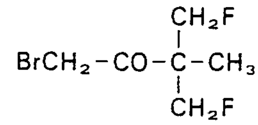

Entsprechend Beispiel 1 durch Umsetzung von 3,3-Bisfluormethylbutan-2-on mit Brom.

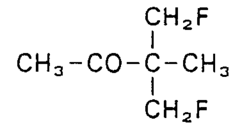

In einem Dreihalskolben mit Rührer, Tropftrichter und Liebigkühler mit gekühlter Vorlage werden 400 ml Tetraäthylenglykol und 46,4 g Kaliumfluorid (0,8 mol) vorgelegt und auf 170°C aufgeheizt. Man legt an den Vorstoss des Liebigkühlers ein Wasserstrahlvakuum (Druck ca. 20 bis 30 mbar) an. Dann werden während 45 min 57,6 g (0,2 mol) 2-Acetyl-2-methylpropan-1,3-diolbismethansulfonat, gelöst in 100 ml Teträthylenglykol, zugetropft. Das entstehende 3,3-Bisfluormethylbutan-2-on wird während der Reaktion in die gekühlte Vorlage abdestilliert. Nach dem Zutropfen wird noch während 1 h bei 175°C weiter destilliert.

Das aufgefangene Destillat wird anschliessend redestilliert. Man erhält 14 g (ca. 51,5% der Theorie) 3,3-Bisfluormethylbutan-2-on vom Siedepunkt 43 bis 46°C/12 mm Hg-Säule.

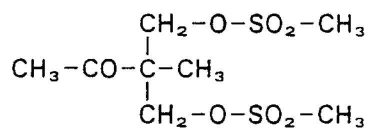

66 g (0,5 mol) 3-Oxa-2,2-bis-(hydroxymethyl)-butan (zur Herstellung vgl. Beilstein *H 1*, E III 3306, IV 4132 und J. Chem. Soc., London, *1932*, 2671) werden in 300 ml 1,2-Dichloräthan gelöst, 114,5 g (1 mol) Methansulfonsäurechlorid zugetropft und bei 0 bis 5°C 158 g (2 mol) Pyridin zugetropft. Man lässt 15 h bei Raumtemperatur nachrühren und gibt dann den Ansatz auf 600 ml Eiswasser und 100 ml konz. Salzsäure. Dabei fällt ein Feststoff aus, der abgesaugt wird. Die wässrige Phase wird mit 1000 ml Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Äther suspendiert. Der Rückstand wird abgesaugt und mit 100 ml Äther gewaschen.

Man erhält 100 g (ca. 70% der Theorie) 2-Acetyl-2-methylpropan-1,3-diol-bis-methansulfonat vom Schmelzpunkt 105 bis 108°C.

In entsprechender Weise werden die nachfolgenden Beispiele der allgemeinen Formel I

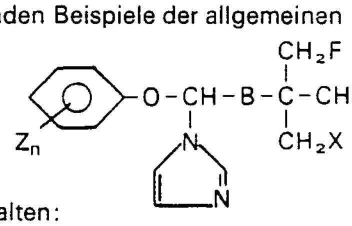 (I)

erhalten:

| Bsp. Nr. | Z | B | X | Schmelzpunkt (°C) bzw Siedepunkt (°C)/mm Hg-Saule |
|---|---|---|---|---|
| 4 | 4-Cl | CO | H | Ol |
| 5 | 4-Cl | CO | H | 260-65 (× ½ NDS) |
| 6 | 4-Br | CO | H | 142 (× HCl) |
| 7 | 4-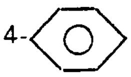 | CO | F | Ol |
| 8 | 4-Cl | CO | F | 250 (× ½ NDS) |
| 9 | 4-F | CO | F | 245-50 (× ½ NDS) |
| 10 | 4-Cl | CH(OH) | H | 148-52 |
| 11 | 4-Br | CH(OH) | H | 160-62 |
| 12 | 4-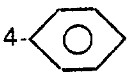 | CH(OH) | F | 151 |
| 13 | 4-Cl | CH(OH) | F | 137 |
| 14 | 2-Cl | CO | F | 206-10 (× ½ NDS) |
| 15 | 2,3-$Cl_2$ | CO | F | 198-202 (× ½ NDS) |
| 16 | 2-Cl, 4-$CH_3$ | CO | F | 204 (× ½ NDS) |
| 17 | 2,4-$Cl_2$ | CH(OH) | F | 106-09 (A-Form) |
| 18 | 2-Cl | CH(OH) | F | 110-19 (A-Form) |
| 19 | 4-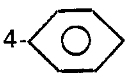 | CO | H | 87-106 |
| 20 | 4-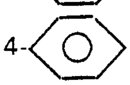 | CH(OH) | H | 120-22 |
| 21 | 4-F | CH(OH) | F | 98-112 |
| 22 | 2,4-$Cl_2$ | CO | F | 124 |
| 23 | 4-Br | CO | F | 260 (× ½ NDS) |
| 24 | 4-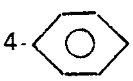-Cl | CO | F | Ol |
| 25 | 3-Cl | CO | F | Ol |
| 26 | 4-Br | CH(OH) | F | 161-63 |
| 27 | 4-Cl, 2-$CH_3$ | CH(OH) | F | 108-15 |
| 28 | 4-$COOC_2H_5$ | CO | F | 243-45 (× ½ NDS) |
| 29 | 4-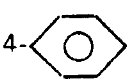-Cl | CH(OH) | F | 151-65 |
| 30 | 3-Cl | CH(OH) | F | 239-43 (× ½ NDS) |
| 31 | 4-$CH_3$ | CH(OH) | F | 143-47 |
| 32 | 4-$NO_2$ | CH(OH) | F | 168-73 |
| 33 | 4-$CH_3$ | CO | F | 260 (× ½ NDS) |
| 34 | 4-$NO_2$ | CO | F | >260 (× ½ NDS) |
| 35 | 4-$COOCH_3$ | CO | F | 250-52 (× ½ NDS) |
| 36 | 2-F | CO | H | 135 (× HCl) |
| 37 | — | CO | H | 153 (× HCl) |
| 38 | — | CH(OH) | H | 137 |
| 39 | 2- | CO | F | 223-25 (× ½ NDS) |
| 40 | 4-CN | CO | F | 256 (× ½ NDS) |
| 41 | 2-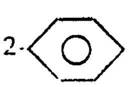 | CH(OH) | F | 97-107 |
| 42 | 4-$COOCH_3$ | CH(OH) | F | 135-47 |
| 43 | 3-Br | CO | F | 245 (× ½ NDS) |
| 44 | 4-$COOC_2H_5$ | CH(OH) | F | 123-27 |
| 45 | 4-CN | CH(OH) | F | 107-17 |
| 46 | 3,4-$Cl_2$ | CO | H | 156-60 (Zers.) (× HCl) |

NDS: 1,5-Naphthalindisulfonsäure; A-Form = eine der beiden möglichen geometrischen Isomeren

## Patentansprüche

1. Fluorierte 1-Imidazolylbutanderivate der allgemeinen Formel I

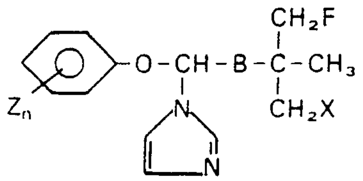

(I)

in welcher

B für die Ketogruppe oder die CH(OH)-Gruppierung steht,

X für Wasserstoff oder Fluor steht,

Z für Halogen, Alkyl mit 1 bis 4 Kohlenstoffatomen, Nitro, Cyano, Alkoxycarbonyl mit 1 bis 4 Kohlenstoffatomen im Alkylteil oder gegebenenfalls durch Halogen substituiertes Phenyl steht und

n für die Zahlen 0, 1, 2 oder 3 steht,

sowie deren Säureaddukte oder Metallsalzkomplexe.

2. Verfahren zur Herstellung von fluorierten 1-Imidazolylbutanderivaten der allgemeinen Formel I

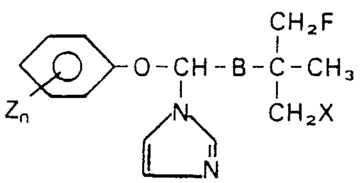

(I)

in welcher

B für die Ketogruppe oder die CH(OH)-Gruppierung steht,

X für Wasserstoff oder Fluor steht,

Z für Halogen, Alkyl mit 1 bis 4 Kohlenstoffatomen, Nitro, Cyano, Alkoxycarbonyl mit 1 bis 4 Kohlenstoffatomen im Alkylteil oder gegebenenfalls durch Halogen substituiertes Phenyl steht und

n für die Zahlen 0, 1, 2 oder 3 steht,

sowie von deren Säureaddukten oder Metallsalzkomplexen, dadurch gekennzeichnet, dass man in an sich bekannter Weise Halogenätherketone der allgemeinen Formel II

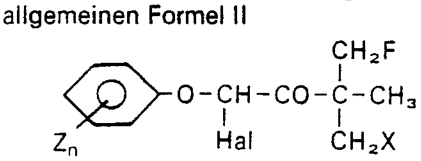

(II)

in welcher

X, Z und n die obenangegebene Bedeutung haben und

Hal für Halogen steht,

mit Imidazol in Gegenwart eines Säurebindemittels und gegebenenfalls in Gegenwart eines Verdünnungsmittels umsetzt und

gegebenenfalls noch die so erhaltenen Ketoderivate nach bekannten Methoden in üblicher Weise reduziert, und an die so erhaltenen Verbindungen der allgemeinen Formel I gegebenenfalls anschliessend eine Säure oder ein Metallsalz addiert.

3. Fungizide Mittel für den Pflanzenschutz, gekennzeichnet durch einen Gehalt an mindestens einem fluorierten 1-Imidazolylbutanderivat der allgemeinen Formel I in Ansprüchen 1 und 2.

4. Verwendung von fluorierten 1-Imidazolylbutanderivaten der allgemeinen Formel I in Ansprüchen 1 und 2 zur Bekämpfung von Pilzen im Pflanzenschutz.

5. Verfahren zur Herstellung von fungiziden Mitteln für den Pflanzenschutz, dadurch gekennzeichnet, dass man fluorierte 1-Imidazolylbutanderivate der allgemeinen Formel I in Ansprüchen 1 und 2 mit Streckmitteln und/oder oberflächenaktiven Mitteln vermischt.

## Claims

1. Fluorinated 1-imidazolylbutane derivates of the general formula I

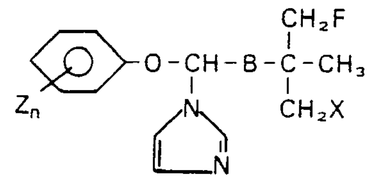

(I)

in which

B represents the keto group or the CH(OH)-grouping,

X represents hydrogen or fluorine,

Z represents halogen, alkyl with 1 to 4 carbon atoms, nitro, cyano, alkoxycarbonyl with 1 to 4 carbon atoms in the alkyl part of phenyl which is optionally substituted by halogen and

n represents the numbers 0, 1, 2 or 3,

and acid adducts or metal salt complexes thereof.

2. Process for the preparation of fluorinated 1-imidazolylbutane derivatives of the general formula I

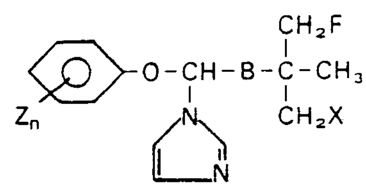

(I)

in which

B represents the keto group or the CH(OH)-grouping,

X represents hydrogen or fluorine,

Z represents halogen, alkyl with 1 to 4 carbon atoms, nitro, cyano, alkoxycarbonyl with 1 to 4 carbon atoms in the alkyl part or phenyl which is optionally substituted by halogen and

n represents the numbers 0, 1, 2 or 3,

and acid adducts or metal salt complexes thereof, characterised in that halogenoether ketones of the general formula II

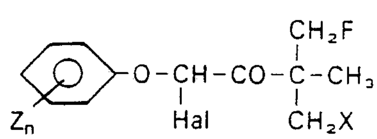

(II)

in which

X, Z and n have the meaning indicated above and

Hal represents halogen,

are reacted, in a manner known per se, with imidazole in the presence of an acid-binding agent and if appropriate in the presence of a diluent and, if required, the resulting keto derivatives are reduced in the customary manner according to known methods and an acid or a metal salt is optionally then added onto the compounds of the general formula I thus obtained.

3. Fungicidal compositions for plant protection, characterised in that they contain at least one fluorinated 1 imidazolylbutane derivative of the general formula I in Claims 1 and 2.

4. Use of fluorinated 1-imidazolylbutane derivatives of the general formula I in Claims 1 and 2 for combating fungi in the protection of plants.

5. Process for the preparation of fungicidal compositions for the protection of plants, characterised in that fluorinated 1-imidazolylbutane derivatives of the general formula I in Claims 1 and 2 are mixed with extenders and/or surface-active agents.

## Revendications

1. Dérivés fluorés de 1-imidazolylbutane de formule générale I

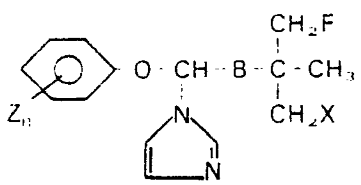

(I)

dans laquelle

B représente le groupe céto ou le groupement CH(OH),

X représente de l'hydrogène ou du fluor,

Z représente de l'halogène, un alcoyle ayant 1 à 4 atomes de carbone, nitro, cyano, alcoxycarbonyle ayant 1 à 4 atomes de carbone dans la portion alcoyle, ou phényle éventuellement substitué par de l'halogène et

n représente les nombres 0, 1, 2 ou 3,

de même que leurs produits d'addition d'acides ou complexes de sels métalliques.

2. Procédé de fabrication de dérivés fluorés de 1-imidazolylbutane de formule générale I

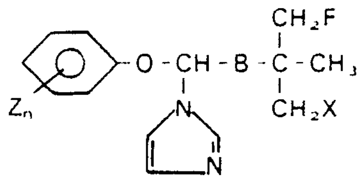

(I)

dans laquelle

B représente le groupe céto ou le groupement CH(OH),

X représente de l'hydrogène ou du fluor,

Z représente de l'halogène, un alcoyle ayant 1 à 4 atomes de carbone, nitro, cyano, alcoxycarbonyle ayant 1 à 4 atomes de carbone dans la portion alcoyle, ou phényle éventuellement substitué par de l'halogène et

n représente les nombres 0, 1, 2 ou 3,

de même que leurs produits d'addition d'acides ou complexes de sels métalliques, caractérisé en ce que de manière connue en elle-même on fait réagir des haloéthercétones de formule générale II

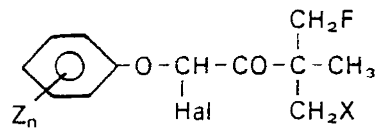

(II)

dans laquelle

X, Z et n ont la signification indiquée plus haut et

Hal représente de l'halogène,

avec de l'imidazol en présence d'un agent fixateur d'acide et éventuellement en présence d'un diluant,

en ce qu'éventuellement on réduit en outre les dérivés cétoniques ainsi obtenus par des méthodes connues de la manière usuelle et en ce que par la suite on fixe un acide ou un sel métallique sur les composés ainsi obtenus de formule générale I.

3. Agents fongicides pour la protection des plantes, caractérisés par une teneur en au moins un dérivé fluoré de 1-imidazolylbutane de formule générale I des revendications 1 et 2.

4. Utilisation des dérivés fluorés de 1-imidazolylbutane de formule générale I des revendications 1 et 2 pour combattre les champignons dans la protection des plantes.

5. Procédé de fabrication d'agents fongicides pour la protection des plantes, caractérisé en ce qu'on mélange des dérivés fluorés de 1-imidazolylbutane de formule générale I des revendications 1 et 2 avec des diluants et/ou des agents tensioactifs.